# FASCICULE DE BREVET EUROPEEN

(11) **EP 1 663 084 B1**
(45) Date de publication et mention de la délivrance du brevet: **11.03.2009**
(21) Numéro de dépôt: 04787396.3
(22) Date de dépôt: 17.09.2004
(51) Int. Cl.: A61F 5/448

(54) **Raccord de dispositif pour stomie**
Verbindende Ostomie-Vorrichtung
Connecting ostomy device

(30) Priorité: 18.09.2003 FR 0310982; 13.02.2004 FR 0401491
(43) Date de publication de la demande: 07.06.2006
(73) Titulaire: B.Braun Medical SAS, 92100 Boulogne-Billancourt (FR)
(72) Inventeur: SHAN, Nicolas, F-94300 Vincennes (FR); MACQUIN, Alexandre, F-91190 Gif Sur Yvette (FR)
(74) Mandataire: Schmit, Christian Norbert Marie
(86) Numéro de dépôt international: PCT/FR2004/002360
(87) Numéro de publication internationale: WO 2005/025466

(56) Documents cités:
- EP-A- 0 598 625
- EP-A- 0 747 026
- DE-U- 29 915 282
- FR-A- 2 396 541
- US-A- 4 636 205
- US-A- 4 846 820

## Description

L'invention concerne des dispositifs pour stomie ayant une construction en deux parties.

Les dispositifs pour stomie en deux parties comportent un dispositif de fixation ou support, qui est collé à la peau autour d'une stomie, et une poche amovible destinée à être raccordée au dispositif de fixation. L'avantage de ces dispositifs en deux parties est qu'un seul collage d'un dispositif de fixation autour de la stomie permet l'utilisation successive de plusieurs poches, en général trois au moins. En effet, bien qu'on sache réaliser maintenant des matériaux constituant des gommes capables d'absorber l'humidité et non allergéniques, l'opération de décollage soumet toujours la peau à des contraintes qu'il est souhaitable d'espacer dans le temps.

On a donc réalisé divers raccords destinés à fixer de manière étanche une poche de recueil à un dispositif de fixation. Il existe essentiellement deux types génériques de tels raccords, un type mécanique et un type adhésif.

Les raccords de type mécanique existent avec de nombreuses variantes. On en décrit trois qui sont représentatifs de cette diversité.

Dans un premier exemple de raccord mécanique, un dispositif de fixation ayant une rondelle de gomme porte un anneau ayant plusieurs lèvres délimitant un canal dans lequel vient se loger une joue d'un anneau porté par une poche. La tenue mécanique est assurée par la coopération de lèvres des deux anneaux, l'étanchéité est assurée par contact d'une lèvre de l'anneau avec le corps de l'anneau, est le guidage pour le positionnement est assuré lorsqu'un anneau vient se loger sur l'autre anneau, l'enclenchement étant assuré simplement par application d'une force aux anneaux.

Un second dispositif de raccord mécanique connu est tel qu'un dispositif de fixation et une poche comportent chacun un anneau, les anneaux ayant des lèvres destinées à s'imbriquer mutuellement pour assurer à la fois la tenue mécanique et l'étanchéité.

Un troisième raccord mécanique connu possède un petit levier pivotant qui fait tourner un dispositif de blocage qui, une fois deux anneaux enclenchés l'un sur l'autre, les bloque par serrage pour assurer une tenue très robuste.

Tous ces dispositifs mécaniques connus présentent l'avantage de ne permettre la mise en coopération de deux anneaux que lorsqu'ils sont bien centrés l'un sur l'autre, si bien que le positionnement des deux parties de raccord est excellent.

Ils présentent cependant des inconvénients. L'étanchéité est obtenue par contact d'une ou plusieurs lèvres avec une surface complémentaire. Pour qu'une lèvre donne une bonne étanchéité, il faut qu'elle ait une bonne élasticité, c'est-à-dire qu'elle doit être soit très mince, soit formée d'un matériau relativement souple. Si le matériau de l'anneau, et donc de la lèvre, est souple, la résistance mécanique obtenue est réduite. Si le matériau de l'anneau est rigide et dur, l'ensemble du raccord constitue un élément rigide qui crée un inconfort certain.

En outre, tous les éléments à lèvre doivent respecter des tolérances serrées ; ils sont formés par injection et sont donc croûteux.

Compte tenu des inconvénients de ces raccords mécaniques, on a essayé d'utiliser des raccords fonctionnant par collage. On a donc réalisé des raccords dans lesquels une rondelle adhésive solidaire de la poche, protégée par un papier protecteur, est collée sur une piste d'un dispositif de fixation à l'utilisateur. Les avantages d'un tel raccord par collage sont évidents : l'ensemble peut être souple et peu épais, il peut donc donner un bon confort, et, comme la surface de collage peut être étendue, l'étanchéité et la tenue mécanique peuvent être excellentes.

Cependant, ce raccord par collage pose un problème : il est difficile de positionner une partie adhésive de la poche sur le dispositif de fixation en assurant l'alignement des orifices de ces deux éléments.

Etant donné la nature très adhésive de la partie de raccord de la poche, dès qu'elle a commencé à adhérer sur une partie antagoniste, il n'est plus possible de déplacer les deux parties l'une par rapport à l'autre. En outre, si les deux parties de raccord sont souples, et donnent donc un avantage au point de vue du confort, l'application uniforme des deux parties l'une sur l'autre présente des difficultés dues à cette souplesse même.

On connaît déjà, d'après le document FR-2 396 541 un raccord de poche de stomie tel qu'un dispositif de fixation comporte un embout circulaire dépassant du dispositif autour de son orifice et entouré d'une piste de collage et, à l'extérieur de celle-ci, d'organes de fixation à boucles et crochets. La poche a une partie de raccord qui comporte une surface adhésive circulaire entourant un orifice et, autour de cette surface adhésive, des organes de fixation à boucles et crochets. Lors de l'utilisation, l'embout du dispositif de fixation est introduit dans l'orifice de la poche avant que la surface adhésive ne vienne au contact de la piste de collage ou que les organes de fixation à boucles et crochetas ne viennent en coopération. Les orifices sont donc bien alignés. Un premier inconvénient de ce dispositif est que l'embout, pour être efficace doit avoir une certaine longueur : il donne alors une grande rigidité à la partie de fixation, donc un grand inconfort. Surtout, comme la surface adhésive entoure l'embout, le papier qui la protège doit être retiré avant que l'embout ne vienne dans l'orifice de la poche. L'extrémité de l'embout vient donc obligatoirement au contacte de la surface adhésive dégagée avant de pénétrer dans l'orifice de la poche. Si l'adhésif est très efficace, il est presque impossible de le décoller sans détériorer gravement l'adhésif : c'est sans doute la raison pour laquelle la fonction de retenue mécanique de la poche n'est pas assurée par la surface adhésive, mais par les organes de fixation à boucles et crochets.

L'invention a pour objet la solution de ce problème de positionnement mutuel des deux parties de raccord dans le cas d'un collage.

Plus précisément, selon l'invention, la partie de fixation et la poche peuvent d'abord être fixées au moins partiellement l'une à l'autre avant que la surface adhésive ne soit séparée de son papier protecteur. Ainsi, lorsque le papier protecteur est retiré, le dispositif de fixation et la poche ne peuvent plus prendre qu'une position dans laquelle leurs orifices sont alignés.

Ainsi, l'invention concerne un raccord de dispositif pour stomie, destiné à raccorder une poche de recueil à un dispositif de fixation destiné à être collé à la peau d'un utilisateur, le raccord ayant pour fonctions la transmission du poids de la poche de recueil au dispositif de fixation, le positionnement de la poche par rapport au dispositif de fixation, afin qu'un orifice du dispositif de fixation soit, en communication avec un orifice de la poche de recueil, et l'étanchéité entre la poche de recueil et le dispositif de fixation, le raccord comportant une première partie de raccord solidaire du dispositif de fixation et une seconde partie de raccord solidaire de la poche de recueil, l'une des parties de raccord ayant une surface adhésive couverte avant utilisation par une feuille protectrice amovible et l'autre partie de raccord ayant une piste d'adhérence, si bien que les deux parties de raccord peuvent coopérer l'une avec l'autre par collage dans un plan pratiquement perpendiculaire aux axes des orifices de la poche et du dispositif de fixation, et que la transmission du poids de la poche de recueil et l'étanchéité entre le dispositif de fixation et la poche de recueil sont assurées par collage ; la première partie et la seconde partie du raccord comportant des organes complémentaires de fixation destinés à limiter les possibilités de déplacement relatif de la poche et du dispositif de fixation afin que les deux parties de raccord n'aient pratiquement qu'une position relative possible lorsqu'elles sont adjacentes, cette position correspondant à l'alignement des orifices du dispositif de fixation et de la poche de recueil. Selon l'invention, les organes complémentaires de fixation forment un dispositif d'articulation délimitant un axe de pivotement distant des orifices du dispositif de fixation et de la poche de recueil, la feuille protectrice étant pratiquement entièrement d'un seul côté de l'axe de pivotement, de manière à pouvoir être retirée de la surface adhésive après que les organes complémentaires de fixation ont été mis en coopération.

Dans un autre mode de réalisation, les organes complémentaires de fixation comportent au moins deux boutons-pression alignés sur un axe.

Dans un autre mode de réalisation, les organes complémentaires de fixation comportent deux éléments solidaires respectivement du dispositif de fixation et de la poche et pouvant assurer une fixation mutuelle par attraction magnétique.

Dans un autre mode de réalisation, les organes complémentaires de fixation comportent une languette solidaire d'une première partie du raccord et un arceau solidaire de l'autre partie du raccord.

Dans un autre mode de réalisation, les organes complémentaires de fixation comportent une patte relativement rigide, solidaire d'une partie du raccord et destinée à s'enclencher dans un logement solidaire de l'autre partie de raccord.

Dans un autre mode de réalisation, les organes complémentaires de fixation comportent des parties conformées de tissu à boucles et à crochets solidaires de chacune des parties de raccord.

Dans un autre mode de réalisation, les organes complémentaires de fixation comportent au moins un premier organe disposé sur une première partie de raccord, et plusieurs seconds organes disposés sur l'autre partie de raccord et ayant des positions espacées angulairement autour de l'orifice de la partie correspondante de raccord, le premier organe pouvant coopérer avec l'un quelconque des seconds organes. Par exemple, la partie portant la languette peut comporter plusieurs languettes ayant des positions espacées angulairement autour de son orifice.

Dans une variante, le raccord comporte des organes auxiliaires de fixation des parties de raccord.

Dans un autre mode de réalisation, la feuille protectrice de la couche adhésive comporte au moins deux parties pliées en portefeuille dont les plis sont adjacents sur la surface adhésive, de sorte que cette surface adhésive est totalement couverte, et le volet qui n'est pas collé à la surface adhésive possède une patte de saisie dépassant au-delà des limites de la surface adhésive.

Dans un autre mode de réalisation, le raccord comprend un dispositif de retenue auxiliaire des parties de raccord par emboîtement.

De préférence, le dispositif de fixation comporte une rondelle souple ayant un orifice pratiquement central destiné à entourer une stomie, la rondelle étant destinée à être collée à la peau d'un utilisateur autour d'une stomie, et une piste fixée à la rondelle de manière étanche autour de l'orifice.

Selon des variantes avantageuses, la partie ayant la surface adhésive étant destinée à être décollée suivant une direction après utilisation, la configuration de cette surface adhésive est telle que la longueur totale du front de décollage par pelage, en direction générale perpendiculaire à la direction de décollage, est modulée afin qu'elle soit sensiblement constante sur la plus grande partie de la longueur de la partie ayant la surface adhésive dans la direction de décollage.

Selon des variantes, la partie ayant la surface adhésive est totalement couverte de colle, ou la partie ayant la surface adhésive n'est que partiellement couverte de colle.

D'autres caractéristiques et avantages de l'invention seront mieux compris à la lecture de la description qui va suivre d'exemples de réalisation, faite en référence aux dessins annexés sur lesquels:
- la figure 1 représente en perspective un raccord de stomie selon l'invention dans une étape initiale de mise en place, les parties de la poche autres que le raccord étant omises pour la clarté de la représentation;
- la figure 2 représente en perspective le raccord de stomie de la figure 1 dans une étape ultérieure de mise en place ;
- la figure 3 représente en perspective le raccord de stomie de la figure 1 une fois réalisée la mise en place ;
- les figures 4a et 4b représentent respectivement un dispositif de fixation et une poche dans lesquels les organes complémentaires de fixation sont des boutons-pression ;
- les figures 5a et 5b représentent respectivement un dispositif de fixation et une poche dans lesquels les organes complémentaires de fixation sont des éléments magnétiques ;
- les figures 6a et 6b représentent respectivement un dispositif de fixation et une poche dont les organes complémentaires de fixation sont formés par une attache à éléments mâle et femelle ;
- les figures 7a et 7b représentent respectivement un dispositif de fixation et une poche dans lesquels les organes complémentaires de fixation sont des éléments de fixation à boucles et crochets, et la feuille protectrice de la couche adhésive comporte deux parties pliées en portefeuille ;
- les figures 8a et 8b représentent respectivement un dispositif de fixation et une poche dans lesquels les organes complémentaires de fixation, sont des éléments de fixation à boucles et crochets fixés au dispositif de fixation et au corps de la poche elle-même, et la feuille protectrice de la couche adhésive comporte deux parties pliées en portefeuille ;
- la figure 9 représente un raccord de stomie analogue à celui des figures 1 à 3, une fois réalisée la mise en place, mais comportant un organe supplémentaire de fixation constitué d'un bec s'accrochant à l'orifice de la partie de raccord solidaire de la poche ;
- la figure 10 représente sous forme agrandie le bec représenté sur la figure 9 ;
- les figures 11a et 11b représentent respectivement un dispositif de fixation et une poche dans lesquels les organes complémentaires de fixation sont des rondelles emboîtables solidaires l'une du dispositif de fixation et l'autre de la poche ;
- la figure 12 représente un élément de liaison qui peut être la rondelle à surface adhésive, solidaire de la poche des figures 1 à 3 ;
- la figure 13 est un graphique représentant la variation de la longueur totale du front de pelage, en direction perpendiculaire à une direction T de décollage, en fonction de la position sur la face de l'élément de liaison, et elle représente aussi la variation de la force de retenue au décollage ;
- la figure 14 représente la surface de collage d'un élément de liaison ayant une forme définie selon l'invention ;
- la figure 15 représente la variation de la force de retenue au décollage de l'élément de liaison selon l'invention représenté sur la figure 14 ;
- la figure 16 représente la surface de collage d'un autre élément de liaison ayant une forme définie selon l'invention ; et
- la figure 17 représente une poche de stomie munie de l'élément de liaison de la figure 16.

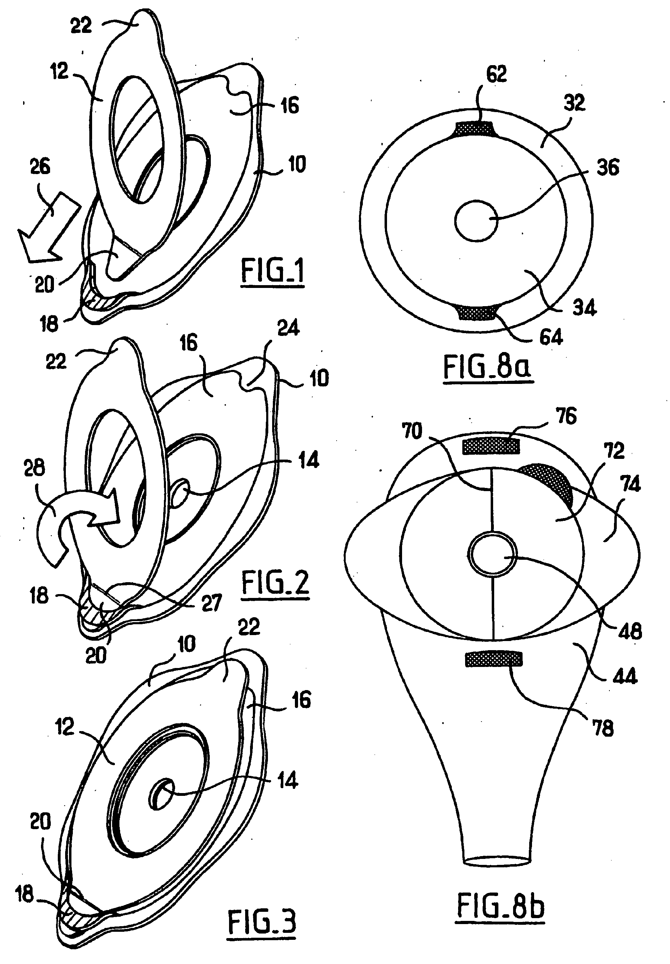

Le raccord de stomie représenté sur les figures 1 à 3 comporte une partie solidaire d'un dispositif de fixation 10 et une autre partie de raccord 12 associée à une poche (non représentée).

Le dispositif de fixation 10 comporte une rondelle de gomme et une piste 16. De manière connue, la gomme contient un polymère et des hydrocolloïdes, et elle permet une fixation robuste à la peau de l'utilisateur autour d'une stomie.

Du côté opposé à la partie adhésive de la gomme, la piste 16 est fixée à la rondelle autour d'un orifice 14. Cette piste constitue une première partie de raccord. Elle comporte une partie plane et souple et, à une partie de sa périphérie, un arceau 18. Elle est avantageusement formée de polyéthylène, d'un copolymère d'éthylène et d'acétate de vinyle, ou d'un polymère analogue qui peut être aisément moulé pour la formation de l'arceau 18 et soudé à la rondelle de gomme, autour de l'orifice 14 et sur une partie seulement de la distance comprise entre le bord de l'orifice et la périphérie de la piste qui est donc libre.

La partie 12 de raccord de la poche entoure un orifice et possède une languette 20. La face de la partie 12 qui n'apparaît pas sur les figures 1 à 3 est revêtue d'un adhésif, lui-même revêtu d'une feuille protectrice, par exemple un papier protecteur siliconé. Une patte 22 permet la tenue de la partie 12 de raccord lors du décollage. De préférence, la piste 16 possède, en face de l'emplacement que doit occuper la patte 22, une encoche 24 formant un dégagement qui facilite la saisie de la patte entre deux doigts.

On décrit maintenant l'utilisation du raccord de stomie en référence aux figures 1 à 3. L'utilisateur, sur lequel est collé le dispositif de fixation 10, saisit une poche munie de la partie 12 de raccord qui porte, sur toute sa face adhésive, un papier protecteur, de préférence coupé entre la languette 20 et le reste de la partie 12 de raccord. Il introduit la languette 20 sous l'arceau 18, comme l'indique la flèche 26. Il peut effectuer l'opération progressivement et la recommencer autant de fois qu'il le souhaite jusqu'à ce que la languette ait totalement pénétré sous l'arceau. A ce moment, la partie 12 de raccord ne peut plus être déplacée que par pivotement autour de l'axe 27 qui sépare la languette du reste de la partie 12.

A ce moment, l'utilisateur qui tient la poche d'une main saisit avec son autre main le papier protecteur, qui a lui-même de préférence une patte qui dépasse (non représentée). Il retire alors ce papier par pelage. Il ne lui reste plus qu'à faire pivoter la partie 12 de raccord qui vient se coller en position parfaitement alignée sur la piste 16.

La caractéristique essentielle est qu'il est possible de placer les deux parties de raccord dans une position qui assure l'alignement des orifices avant de retirer le papier protecteur, de sorte que la surface adhésive ne peut venir se coller que sur la piste 16.

Les personnes qui doivent porter de telles poches de stomie souhaitent parfois orienter différemment le corps de poche selon que celle-ci est portée dans la journée (personne debout ou assise) ou la nuit (personne couchée). Le raccord décrit en référence aux figures 1 à 3 ne permet qu'une seule orientation.

Dans une variante (non représentée), la partie 12 de raccord de la poche comporte plusieurs languettes, par exemple trois espacées à des intervalles de 45° ou 60°. Ainsi, la poche peut être orientée vers le bas dans la journée, et vers la droite ou la gauche la nuit.

On note que la surface de collage est très étendue puisqu'elle couvre toute la surface comprise entre l'orifice central et la périphérie circulaire de la piste 16 ou de la partie de raccord 12. Ce collage assure de manière connue une très bonne étanchéité et une très bonne tenue mécanique.

Ces propriétés sont obtenues parce que la piste 16 et la partie de raccord 12 sont parfaitement centrées. On conçoit facilement que, si les deux parties étaient décentrées, d'une part la surface de collage, et donc de tenue mécanique, serait réduite, et d'autre part, la probabilité de fuite au niveau de la partie collée de plus faible largeur serait élevée.

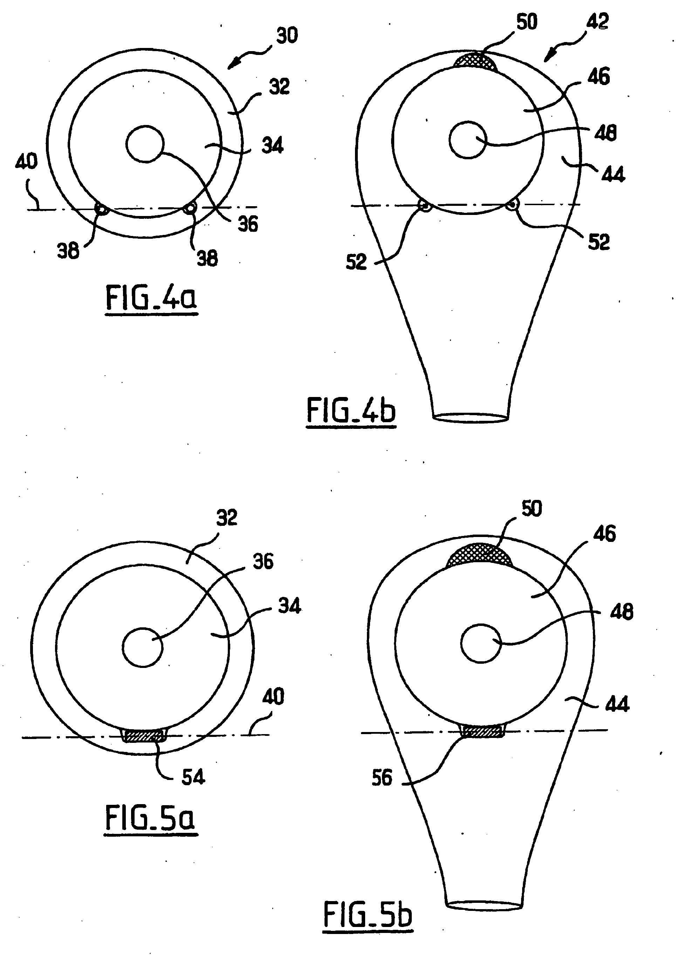

Les figures 4a et 4b représentent, dans un autre mode de réalisation, un dispositif pour stomie comprenant respectivement un dispositif de fixation 30 et une poche 42, qui peuvent être reliés de façon étanche par un raccord selon un autre mode de réalisation de l'invention.

Le dispositif de fixation 30 comporte une rondelle 32 de gomme. De manière connue, cette gomme contient un polymère et des hydrocolloïdes, et elle permet une fixation robuste à la peau de l'utilisateur autour d'une stomie.

Du côté opposé à la partie adhésive de la gomme 32, une piste 34 est fixée à la rondelle 32 autour de l'orifice 36. Cette piste constitue une première partie de raccord. Elle est avantageusement formée de polyéthylène, de copolymère d'éthylène et d'acétate de vinyle, ou d'un polymère analogue qui peut être aisément soudé à la rondelle 32, autour de l'orifice 36 et sur une partie seulement de la distance comprise entre le bord de l'orifice et la périphérie de la piste qui est donc libre.

La poche 42 a un corps 44 et une partie 46 de raccord qui entoure un orifice 48. Une patte ou oreille 50 permet la tenue de la partie 46 de raccord lors du décollage.

Selon l'invention, des organes complémentaires de fixation sont constitués par deux boutons-pression 38, 52, ayant chacune une partie fixée au dispositif de fixation 30 et une autre partie fixée à la poche 42. Dans le mode de réalisation représenté, les parties femelles des boutons-pression 38 sont fixées au bord de la piste 34 et les parties mâles des boutons-pression 52 sont fixées au bord de la partie 46 de raccord qui forme une surface adhésive. Ainsi, les boutons-pression 38, 38 et 52, 52 délimitent un axe 40 de pivotement.

Lors de la mise en place, le dispositif de fixation ayant déjà été collé à la peau de l'utilisateur, la partie de raccord 46, qui a une surface adhésive protégée par un papier protecteur, est approchée par sa partie inférieure des boutons-pression 38 afin que les parties mâles 52 puissent être agrafées sur les parties femelles 38. L'utilisateur peut facilement mettre les boutons-pression en coopération puisqu'il peut les associer à l'aveugle, par détection au toucher. A partir de ce moment, la partie supérieure de la poche 42 ne peut être déplacée par rapport au dispositif de fixation 30 que par pivotement autour de l'axe 40.

L'utilisateur retire alors le papier protecteur de la partie adhésive de raccord 46 et, par pivotement vers le haut autour de l'axe 40, vient appliquer la surface collante contre la piste 34 du dispositif de fixation. Compte tenu de la rigidité relative de la piste 34 et de la partie de raccord 46, les deux parties viennent se coller en étant parfaitement centrées, c'est-à-dire que l'orifice 36 du dispositif de fixation se trouve juste en face de l'orifice 48 de la poche.

Les figures 5a et 5b représentent un dispositif analogue dans lequel le rôle des boutons-pression 38, 52, est joué par deux petits aimants 54, 56, c'est-à-dire que ceux-ci délimitent un axe de pivotement dans la direction de leur longueur (40 sur la figure). Le résultat obtenu est analogue à celui que donne le dispositif des figures 4a et 4b. On note aussi la présence d'une patte ou oreille 50 de préhension permettant de séparer facilement la poche du dispositif de fixation lorsque la poche doit être séparée.

Sur les figures 5a et 5b, comme sur les figures suivantes, on a utilisé des références identiques à celles des figures 4a et 4b pour désigner les éléments analogues.

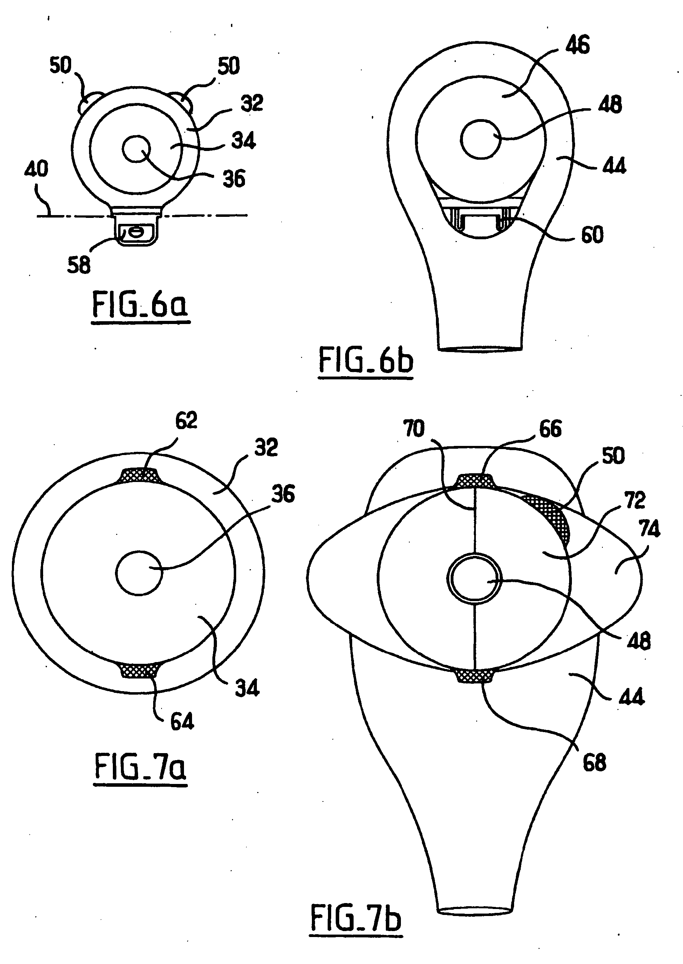

Les figures 6a et 6b représentent un mode de réalisation dans lequel des organes complémentaires de fixation sont constitués par une agrafe élastique 58 destinée à se loger dans un guide 60 délimité par deux rails latéraux. Lorsque la poche 42 doit être placée sur le dispositif de fixation, la partie femelle 60 de guidage destinée à coopérer avec l'agrafe 58 est glissée sur celle-ci et s'enclenche ainsi élastiquement. L'utilisateur entend alors de préférence le bruit d'enclenchement, tel qu'un clic, qui l'avertit que les organes complémentaires de fixation sont en position. Ensuite, par rotation autour de l'axe 40, après enlèvement du papier protecteur de la surface 46, cette surface adhésive est appliquée sur la piste 34 du dispositif de fixation. Dans ce mode de réalisation, on a représenté des pattes de préhension 50 sur le dispositif de fixation.

Bien entendu, l'agrafe peut être remplacée par de nombreux dispositifs ayant des fonctions analogues, tels qu'une pince s'accrochant sur un axe, ou une griffe glissant dans un canal. La caractéristique essentielle est la détermination d'un axe pour la rotation de la poche par rapport au dispositif de fixation.

Dans les modes de réalisation décrits précédemment, la surface adhésive de la poche est amenée contre la surface coopérante de la partie de fixation par rotation autour d'un axe de pivotement décalé latéralement par rapport aux orifices. Il est possible de tenir la partie de poche munie de la surface adhésive éloignée du support de fixation sauf au niveau de l'axe de pivotement au début de la mise en place. Du fait de cet éloignement, dans ces modes de réalisation où l'axe de pivotement est éloigné des orifices, il est même possible d'enlever, totalement ou partiellement, le papier protecteur de la surface adhésive de la poche avant la mise en coopération des organes complémentaires de fixation.

Les figures 7a et 7b représentent un autre mode de réalisation dans lequel le positionnement avant collage est assuré non par rotation autour d'un axe d'articulation, mais par alignement en pratique des deux parties du raccord l'une sur l'autre avant le collage.

Plus précisément, comme l'indiquent les figures 7a et 7b, le dispositif de fixation possède, soit sur la rondelle de gomme 32, soit au bord de la piste 34, deux éléments 62, 64, de fixation du type à boucles et crochets, connus sous le nom commercial "Velcro", qui peuvent être diamétralement opposés comme représenté. La poche comporte, à des emplacements correspondants de la partie de raccord, deux éléments complémentaires de fixation à boucles et crochets 66, 68.

Dans ce cas, comme les éléments de fixation 62, 64 et 66, 68 sont mis en coopération lorsque la surface adhésive de la partie de raccord de la poche n'est pas exposée et est toujours munie de son papier protecteur, il faut que ce papier protecteur puisse être retiré après que les éléments auxiliaires de fixation ont été mis en coopération. En conséquence, le papier protecteur comprend cette fois deux parties séparées pliées en portefeuille suivant un diamètre 70. Chaque partie comporte une partie en demi-cercle 72 disposée sur la moitié de la surface adhésive, au contact de celle-ci, et une autre partie 74 qui lui est raccordée par simple pliage et qui est plus longue afin qu'elle dépasse à l'extérieur de la poche et puisse être saisie. En conséquence, lorsque les éléments de fixation 62, 64 et 66, 68 ont été fixés, les deux parties 74 de papier protecteur dépassent sur les côtés, et il suffit de les tirer pour dégager la surface adhésive qui est alors bien positionnée en face de la piste 34 du dispositif de fixation.

Les figures 8a et 8b représentent une variante dans laquelle les éléments de fixation du type à boucles et crochets 66 et 68, portés par la partie 46 de raccord de la poche sur la figure 7b, sont cette fois placés sur le corps 44 de la poche, comme indiqué par les références 76 et 78. Le fonctionnement est le même.

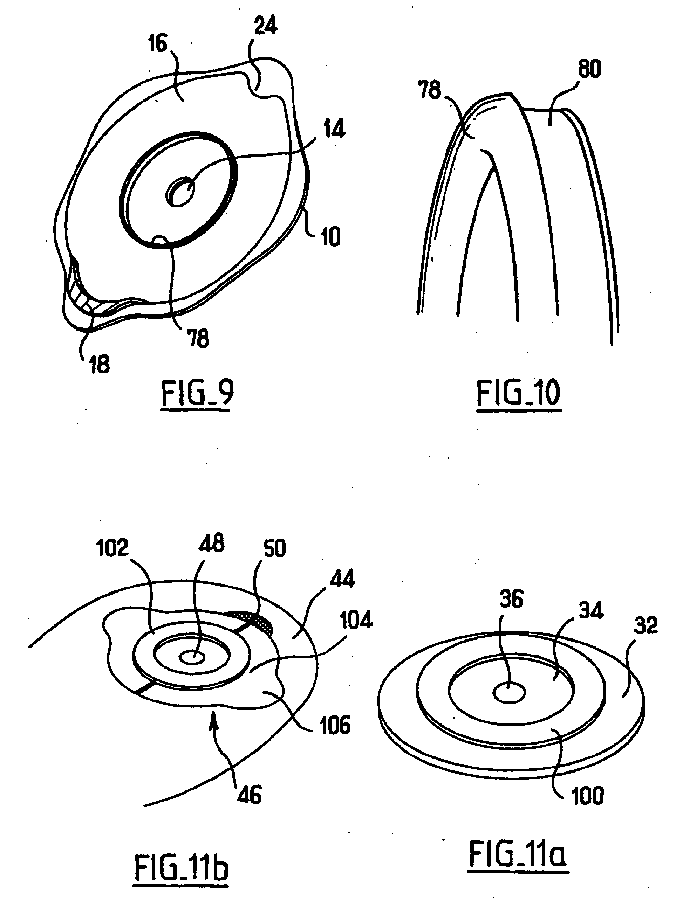

La figure 9 représente une variante supplémentaire. Le dispositif de fixation 10 représenté est analogue à celui des figures 1 à 3, car il comporte une rondelle de gomme à coller sur la peau de l'utilisateur, et une piste 16 qui a un arceau 18 et une encoche 24. La différence porte sur la présence d'un embout 80 solidaire de la rondelle de gomme, portant un bec 78 destiné à s'accrocher au bord de l'orifice de la partie de raccord de la poche, comme représenté plus en détail sur la figure 10.

Cette disposition est très différente du dispositif de la technique antérieure car la longueur de l'embout est très réduite et son matériau est très souple, et il ne crée donc pas un inconfort. Un tel perfectionnement peut être utilisé lorsque la force de fixation par collage est insuffisante, par exemple parce que la surface adhésive est très petite ou parce que la substance adhésive est peu robuste.

Les figures 11a et 11b représentent un autre mode de réalisation dans lequel le dispositif de fixation, qui comporte encore la rondelle de gomme 32, délimitant l'orifice 36, et la piste 34, possède en outre un anneau 100 d'une mousse élastique collée à la périphérie de la piste 34. La partie de raccord 46 porte à sa surface, autour de l'orifice 48, un anneau 102 dont le diamètre externe est très légèrement inférieur ou égal au diamètre interne de l'anneau de mousse 100 du dispositif de fixation. La poche peut donc être facilement positionnée par rapport au dispositif de fixation par logement de l'anneau 102 de dans l'anneau 100 de mousse. Dans ce mode de réalisation, la surface adhésive de la partie de raccord 46 porte un papier protecteur 104 formé de deux parties pliées en portefeuille et munies chacune d'une patte 106. Une fois l'anneau 102 introduit dans l'anneau 100, le papier protecteur 104 est tiré par les pattes 104 et dégage la surface adhésive qui vient se coller, de préférence sur une piste solidaire de la rondelle de gomme 32.

L'anneau 102 peut être d'un type dont les propriétés adhésives sont accrues en présence de liquide aqueux, et peut alors former une zone d'étanchéité secondaire qui protège la zone d'étanchéité principale.

Les divers modes de réalisation décrits précédemment concernent divers aspects dans lesquels l'étanchéité et la tenue mécanique sont essentiellement assurées par le collage.

Dans certains modes de réalisation, la tenue mécanique peut être accrue, par exemple par emboîtement (modes de réalisation des figures 10 et 11a, 11b).

Une propriété essentielle des dispositifs de fixation par collage selon l'invention est le confort donné aux utilisateurs, car le dispositif de fixation comme la poche peuvent être très souples et peuvent s'adapter à la forme du corps.

Bien qu'on ait décrit des modes de réalisation dans lesquels la piste 34 du dispositif de fixation n'est pas adhésive, et la partie de raccord 46 de la poche est adhésive, la disposition peut être inversée. En outre, le collage peut être réalisé avec un adhésif du type à deux composants dont l'un est placé sur la piste et l'autre sur la partie de raccord de la poche.

On n'a pas décrit la réalisation de l'élément protecteur de la surface adhésive, car un tel élément est bien connu. On utilise en général un papier revêtu d'une couche d'un polymère de silicone placée au contact de la surface adhésive. Bien qu'on ait représenté, dans les modes de réalisation des figures 7a, 7b et 8a, 8b, des éléments protecteurs retirés par traction en sens opposés, il est aussi possible que les languettes dépassent dans d'autres directions, par exemple à mi-distance entre l'axe horizontal et un dispositif de fixation à boucles et crochets.

Les organes complémentaires de fixation des dispositifs pour stomie selon l'invention peuvent être utilisés à l'aveugle, sans qu'il soit nécessaire de voir ni le dispositif de fixation ni la poche. Ainsi, l'utilisateur, lorsqu'il a introduit la languette, fixé les boutons-pression ou introduit l'agrafe, peut en toute confiance appliquer la partie adhésive contre la piste sans risquer un défaut d'alignement. L'utilisateur peut alors s'assurer du bon collage par pincement avec les doigts de la piste et de la partie de raccord qui sont collées. Au besoin, l'utilisateur peut s'assurer du positionnement convenable par toucher de la piste et de la partie de raccord dont les périphéries, correspondent. La surface de collage, et donc l'étanchéité, sont alors les meilleures possible.

Dans tous les modes de réalisation décrits, la rondelle portant la surface adhésive de la poche et la piste coopérante forment un élément de liaison qu'on a supposé de forme circulaire, pour des raisons de facilité de fabrication. On a en outre supposé que cette rondelle était entièrement revêtue de substance adhésive. Cependant cette disposition pose un problème à l'utilisation.

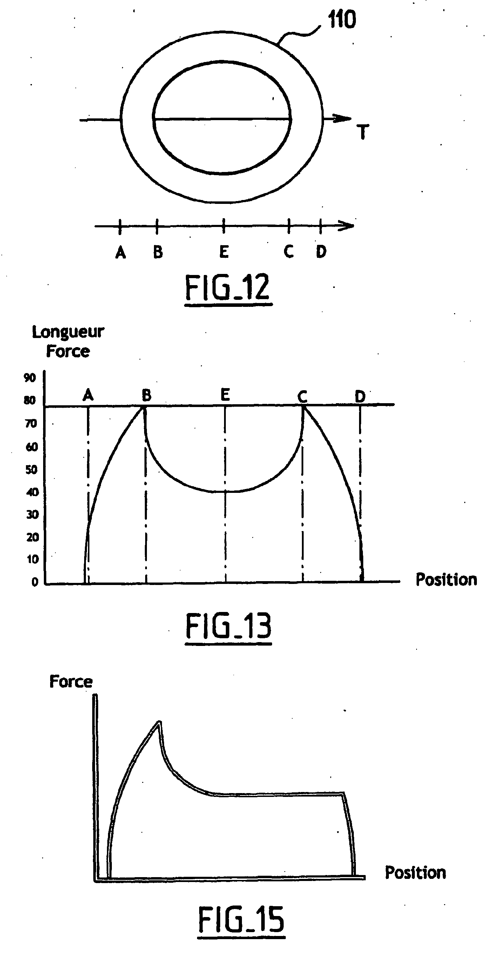

On se réfère à la figure 12 qui représente un tel élément de liaison 110. Lorsque celui-ci a été collé et doit être décollé, une force de décollage est appliquée par la main de l'utilisateur qui se déplace dans la direction T de la figure 12, cette direction étant parallèle au plan de la face à coller de l'élément de liaison et étant appelée dans la suite "direction de décollage". L'élément de liaison se décolle successivement suivant un front linéaire de pelage d'orientation générale perpendiculaire à la direction de décollage T.

L'intersection du front de pelage avec la direction T, à diverses étapes du décollage, est indiquée par les références A, B, E, C et D sur la figure 12, ces références étant reprises sur la figure 13, qui est une courbe représentant la variation de la longueur totale du front de pelage en fonction de la position, portée en abscisses. Dans la suite, cette "longueur totale du front de pelage" est la somme, pour chaque point du front de pelage, des segments de front d'orientation générale perpendiculaire à la direction de décollage et disposés à la surface de l'élément de liaison. La courbe de la figure 13, entre les deux extrémités A et D de l'élément de liaison dans la direction de décollage T, est telle que la longueur totale du front de pelage augmente très rapidement, passe par un premier maximum en B, puis par un minimum en E, puis par un second maximum en C, avant de s'annuler très rapidement en D.

On sait que la force de retenue au décollage d'une surface souple d'adhésivité pratiquement constante est proportionnelle à la longueur du front de pelage de la surface adhésive. Dans le cas de l'élément de liaison de la figure 12, la surface annulaire de l'élément de liaison est totalement couverte de colle d'épaisseur et de composition sensiblement constantes, et la force de retenue au décollage dans la direction T est proportionnelle à la longueur totale du front de pelage. En conséquence, la variation de la force de retenue au décollage est identique à celle qui est représentée sur la figure 13. Elle augmente très rapidement, passe par un premier maximum en B, puis par un minimum en E, puis par un second maximum en C avant de s'annuler très rapidement.

La force de décollage appliquée à un élément de liaison adhésif de poche de stomie est exercée par un être humain. Cette force doit compenser la force de retenue par la colle qui varie comme indiqué sur la figure 13. Lorsque la force de retenue est élevée (en B et C), la main de l'utilisateur doit exercer une force élevée de décollage ; si la force de retenue diminue alors rapidement, comme après le point C de la figure 13, la force de décollage appliquée par la main est tellement supérieure à la force de retenue qu'elle provoque un arrachement brutal. S'il s'agit d'une poche de stomie, la séparation brutale de la poche du corps provoque une souffrance (irritation de la peau) et un secouage de la poche ; elle peut provoquer la projection du contenu à l'extérieur ou même le renversement de la poche.

Dans des modes de réalisation avantageux de l'invention, la longueur totale du front de pelage, au décollage de la surface adhésive de l'élément de liaison, est modulée, afin qu'elle soit sensiblement constante sur la plus grande partie au moins de la longueur de l'élément de liaison dans la direction de décollage.

Plus précisément, dans le cas d'un élément de liaison sensiblement plat recouvert de colle sur une partie au moins d'une de ses faces et destiné à être décollé suivant une direction, la longueur totale du front de décollage par pelage, en direction générale perpendiculaire à la direction de décollage, est modulée afin qu'elle soit sensiblement, constante sur la plus grande partie de la longueur de l'élément de liaison dans la direction de décollage.

Dans un mode de réalisation, la face de l'élément de liaison est totalement couverte de colle, la forme de l'élément de liaison étant adaptée pour donner la modulation voulue de la longueur totale du front de pelage.

Dans un autre mode de réalisation, la face de l'élément de liaison est partiellement couverte de colle, et c'est la forme de la surface adhésive qui est adaptée pour donner la constance voulue de la longueur totale du front de pelage.

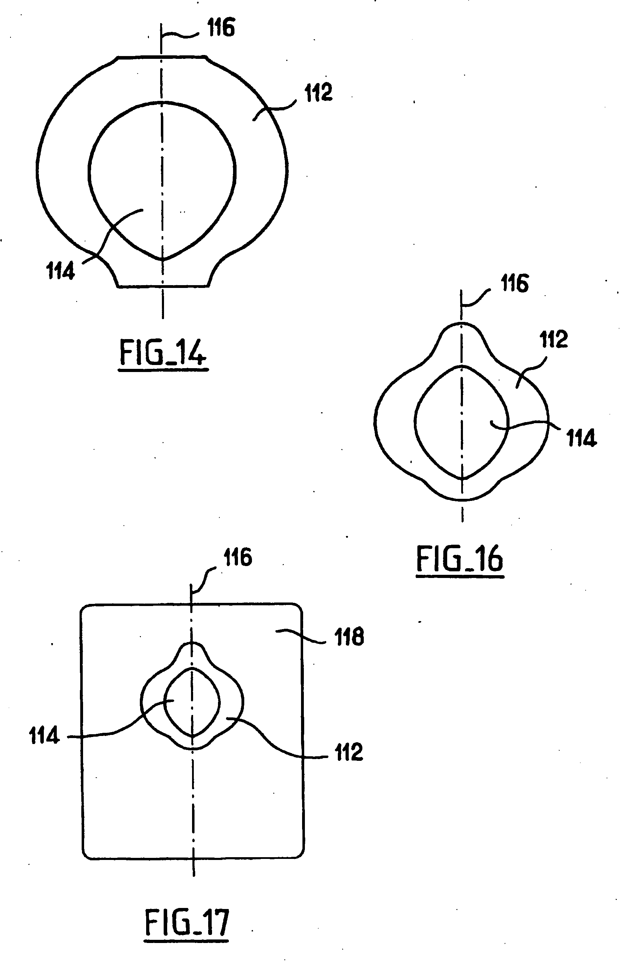

Sur la figure 14, on a représenté la face adhésive d'un élément de liaison 112 selon l'invention, qui est constitué de préférence d'une composition à base de matière thermoplastique, et a un orifice 114. Cette face de l'élément de liaison 112, est entièrement recouverte d'une épaisseur sensiblement constante de colle. L'élément de liaison 112 a deux parties symétriques par rapport à son axe médian 116. La dimension de chacune des deux parties, mesurée en direction perpendiculaire à l'axe 116, est sensiblement constante sur toute la longueur de l'élément de liaison, dans la direction de l'axe médian, sauf aux extrémités.

La longueur totale du front de pelage pendant un décollage est modulée afin qu'elle augmente d'abord rapidement vers une valeur maximale, puis diminue vers une valeur qui reste constante presque jusqu'à l'autre extrémité lorsque la position suivant l'axe 116 varie. Dans cette partie, la vitesse de décollage est constante pour une force appliquée constante, et la partie décollée forme un angle pratiquement constant avec la partie encore collée.

Ce comportement est confirme par le graphique de la figure 15 qui indique que la force à appliquer est d'abord élevée, pour donner un sentiment de sécurité à l'utilisateur, puis diminue rapidement vers une valeur sensiblement constante correspondant à une vitesse de décollage maîtrisée, avant de diminuer à l'extrémité.

La figure 16 représente une variante d'élément de liaison dont la longueur totale du front de pelage devient plus rapidement constante.

Dans le cas d'une poche 118 de stomie telle que représentée sur la figure 17 et qui peut être plus ou moins pleine, le risque de renverser ou de projeter une partie du contenu de la poche est alors réduit.

La poche 118, avantageusement constituée de deux films de matière souple et transparente soudés à leurs bords, par exemple en matière thermoplastique, est partiellement fixée à l'élément de liaison par soudure ou collage.

Dans un autre mode de réalisation, la modulation de la longueur totale du front de pelage, afin qu'il ait une valeur sensiblement constante sur une grande partie de la longueur dans la direction de décollage, est obtenue par application d'un adhésif à l'élément de liaison avec une surface adaptée qui n'est pas identique à celle de l'élément de liaison. Plus précisément, à la surface de l'élément de liaison, il reste des parties dépourvues d'adhésif.

Dans un autre mode de réalisation, la modulation de la longueur totale du front de pelage tient compte d'autres paramètres qui influent sur la force de pelage, tels que la rigidité variable de l'élément de liaison, la présence d'autres éléments solidaires de l'élément de liaison, notamment une bande soudée, etc.

Bien entendu, diverses modifications peuvent être apportées par l'homme de l'art aux raccords qui viennent d'être décrits uniquement à titre d'exemple non limitatif sans sortir du cadre de l'invention.

## Revendications

1. Raccord de dispositif pour stomie, destiné à raccorder une poche de recueil (42) à un dispositif de fixation (30) destiné à être collé à la peau d'un utilisateur,
Le raccord ayant pour fonctions :
la transmission du poids de la poche de recueil(42) au dispositif de fixation (30),
le positionnement de la poche (42) par rapport au dispositif de fixation (30), afin qu'un orifice (36) du dispositif de fixation soit en communication avec un orifice (48) de la poche de recueil, et
l'étanchéité entre la poche de recueil (42) et le dispositif de fixation (30),
le raccord comportant une première partie de raccord (34) solidaire du dispositif de fixation et une seconde partie de raccord (46) solidaire de la poche de recueil,
l'une des parties de raccord ayant une surface adhésive couverte avant utilisation par une feuille protectrice amovible et l'autre partie de raccord ayant une piste d'adhérence, si bien que les deux parties de raccord (14, 34, 46) peuvent coopérer l'une avec l'autre par collage dans un plan pratiquement perpendiculaire aux axes des orifices (14, 36, 48) de la poche et du dispositif de fixation, et que la transmission du poids de la poche de recueil (42) et l'étanchéité entre le dispositif de fixation (30) et la poche de recueil (42) sont assurées par collage,
la première partie et la seconde partie de raccord comportant des organes complémentaires de fixation destinés à limiter les possibilités de déplacement relatif de la poche (42) et du dispositif de fixation (30) afin que les deux parties de raccord n'aient pratiquement qu'une position relative possible lorsqu'elles sont adjacentes, cette position correspondant à l'alignement des orifices (36, 48) du dispositif de fixation (30) et de la poche de recueil (42),
**caractérisé en ce que** les organes complémentaires de fixation forment un dispositif d'articulation délimitant un axe de pivotement (40) distant des orifices du dispositif de fixation et de la poche de recueil, le dispositif d'articulation étant tel que, une fois les organes complémentaires de fixation mis en coopération la poche ne peut être déplacée par rapport au dispositif de fixation que par pivotement autour dudit axe de pivotement, la feuille protectrice étant disposée par rapport à l'axe de pivotement de manière à pouvoir être retirée de la surface adhésive après que les organes complémentaires de fixation ont été mis en coopération.

2. Raccord selon la revendication 1, **caractérisé en ce que** les organes complémentaires de fixation comportent au moins deux boutons-pression (38, 52) alignés sur un axe.

3. Raccord selon la revendication 1, **caractérisé en ce que** les organes complémentaires de fixation comportent deux éléments (54, 56) solidaires respectivement du dispositif de fixation (30) et de la poche (42) et pouvant assurer une fixation mutuelle par attraction magnétique.

4. Raccord selon la revendication 1, **caractérisé en ce que** les organes complémentaires de fixation comportent une languette (20) solidaire d'une première partie du raccord et un arceau (18) solidaire de l'autre partie du raccord.

5. Raccord selon la revendication 1, **caractérisé en ce que** les organes complémentaires de fixation comportent des parties conformées (62, 64, 66, 70, 76, 78) de tissu à boucles et à crochets solidaires de chacune des parties de raccord (34, 46).

6. Raccord selon l'une quelconque des revendications 1 à 5, **caractérisé en ce que** les organes complémentaires de fixation comportent au moins un premier organe (18) disposé sur une première partie de raccord, et plusieurs seconds organes (20) disposés sur l'autre partie de raccord et ayant des positions espacées angulairement autour de l'orifice de la partie correspondante de raccord, le premier organe pouvant coopérer avec l'une quelconque des seconds organes.

7. Raccord selon les revendications 4 et 6 prises ensemble, **caractérisé en ce que** la partie qui comporte la partie portant la languette (20) comporte plusieurs languettes ayant des positions espacées angulairement autour de son orifice.

8. Raccord selon l'une quelconque des revendications précédentes, **caractérisé en ce que** la feuille protectrice (72) de la couche adhésive comporte au moins deux parties pliées en portefeuille dont les plis sont adjacents sur la surface adhésive, de sorte que cette surface adhésive est totalement couverte, et le volet qui n'est pas collé à la surface adhésive possède une patte (74, 106) de saisie dépassant au-delà des limites de la surface adhésive.

9. Raccord selon l'une quelconque des revendications précédentes, **caractérisé en ce que** la partie ayant la surface adhésive est destinée à être décollée suivant une direction (T) après utilisation, et la configuration de cette surface adhésive est telle que la longueur totale du front de décollage par pelage, en direction générale perpendiculaire à la direction de décollage, est modulée afin qu'elle soit sensiblement constante sur la plus grande partie de la longueur de la partie ayant la surface adhésive dans la direction de décollage.

## Claims

1. Ostomy device connection which is intended to connect a collection pouch (42) to a fixing device (30) which is intended to be adhesively-bonded to the skin of a user, the connection serving:
to transmit the weight of the collection pouch (42) to the fixing device (30),
to position the pouch (42) relative to the fixing device (30) so that a hole (36) of the fixing device is in communication with a hole (48) of the collection pouch, and
to provide sealing between the collection pouch (42) and the fixing device (30),
the connection comprising a first connection portion (34) which is fixedly joined to the fixing device and a second connection portion (46) which is fixedly joined to the collection pouch,
one of the connection portions having an adhesive surface which is covered before use by a removable protective sheet and the other connection portion having an adhesion strip, so that the two connection portions (14, 34, 46) can co-operate with each other by means of adhesive-bonding in a plane which is practically perpendicular relative to the axes of the holes (14, 36, 48) of the pouch and the fixing device, and the transmission of the weight of the collection pouch (42) and the sealing between the fixing device (30) and collection pouch (42) are provided by means of adhesive-bonding, the first connection portion and the second connection portion (46) comprising complementary fixing elements which are intended to limit the possibilities for relative displacement of the pouch (42) and the fixing device (30) so that the two connection portions have practically only one possible relative position when they are adjacent, this position corresponding to the alignment of the holes (36, 48) of the fixing device (30) and the collection pouch (42),
**characterised in that that** the complementary fixing elements form an articulation device which delimits a pivot axis (40) remote from the holes of the fixing device (30) and the collection pouch (42), the articulation device being such that when the complementary fixing elements have been brought into co-operation, the collection pouch could only be moved regarding the articulation device by pivoting about said axe, the protective sheet being practically entirely at only one side of the pivot axis, in order to be able to be removed from the adhesive surface after the complementary fixing elements have been brought into co-operation.

2. Connection according to claim 1, **characterised in that** the complementary fixing elements comprise at least two push buttons (38, 52) which are aligned along an axis.

3. Connection according to claim 1, **characterised in that** the complementary fixing elements comprise two elements (54, 56) which are fixedly joined to the fixing device (30) and the pouch (42), respectively, and which are able to provide mutual fixing by means of magnetic attraction.

4. Connection according to claim 1, **characterised in that** the complementary fixing elements comprise a tongue (20) which is fixedly joined to a first portion of the connection and a curved member (18) which is fixedly joined to the other portion of the connection.

5. Connection according to claim 1, **characterised in that** the complementary fixing elements comprise shaped portions (62,64, 66, 70, 76, 78) of hook and loop type fabric which are fixedly joined to each of the connection portions (34, 46).

6. Connection according to any one of claims 1 to 6, **characterised in that** the complementary fixing elements comprise at least a first element (18) which is arranged an a first connection portion, and a plurality of second elements (20) which are arranged an the other connection portion and which have positions which are angularly spaced-apart around the hole of the corresponding connection portion, the first element being able to co-operate with any one of the second elements.

7. Connection according to claims 4 and 6, taken in combination, **characterised in that** the portion which comprises the portion carrying the tongue (20) comprises a plurality of tongues having positions which are angularly spaced-apart around the hole thereof.

8. Connection according to any one of the preceding claims, **characterised in that** the protective sheet (72) of the adhesive layer comprises at least two portions which are folded in the form of a folder whose folds are adjacent an the adhesive surface so that this adhesive surface is completely covered and the flap which is not adhesivelybonded to the adhesive surface has a gripping lug (74, 106) which extends beyond the limits of the adhesive surface.

9. Connection according to any one of the preceding claims, **characterised in that** the portion having the adhesive surface is intended to be detached in one direction (T) after use, and the configuration of this adhesive surface is such that the total length of the face for detaching by means of peeling, in a direction which is generally perpendicular relative to the detaching direction, is modulated so that it is substantially constant over the majority of the length of the portion having the adhesive surface in the detaching direction.

## Patentansprüche

1. Anschluss einer Vorrichtung für eine Stomie, der dazu bestimmt ist, einen Sammelbeutel (42) an eine Befestigungsvorrichtung (30) anzuschließen, die dazu bestimmt ist, auf die Haut eines Benutzers geklebt zu werden,
Der Anschluss erfüllt dabei folgende Funktionen:
Die Übertragung des Gewichtes des Sammelbeutels (42) auf die Befestigungsvorrichtung (30),
die Positionierung des Beutels (42) im Verhältnis zur Befestigungsvorrichtung (30), damit eine Öffnung (36) in der Befestigungsvorrichtung und eine Öffnung (48) im Sammelbeutel zusammenpassen, und
die Abdichtung zwischen dem Sammelbeutel (42) und der Befestigungsvorrichtung (30),
der Anschluss enthält dabei einen ersten Anschlussabschnitt (34), der fest an der Befestigungsvorrichtung anhaftet und einen zweiten Anschlussabschnitt (46), der fest am Sammelbeutel anhaftet,
einer der Anschlussabschnitte weist dabei eine Klebefläche auf, die vor der Verwendung durch eine abziehbare Schutzfolie abgedeckt ist, und der andere Anschlussabschnitt weist eine Haftleiste auf, sodass die beiden Anschlussabschnitte (14, 34, 46) durch Verkleben auf einer Ebene, die praktisch normal zu den Öffnungsachsen (14, 36, 48) des Beutels und der Befestigungsvorrichtung steht, zusammenwirken können, und die Übertragung des Gewichtes des Sammelbeutels (42) sowie die Dichtheit zwischen der Befestigungsvorrichtung (30) und dem Sammelbeutel (42) durch Verkleben gewährleistet werden,
der erste und der zweite Anschlussabschnitt enthalten dabei ergänzende Befestigungsorgane, die dazu bestimmt sind, die Möglichkeiten für eine relative Bewegung zwischen dem Beutel (42) und der Befestigungsvorrichtung (30) einzuschränken, damit die beiden Anschlussabschnitte praktisch nur eine mögliche Position zueinander aufweisen, wenn sie aneinander liegen, wobei diese Position der Flucht zwischen den Öffnungen (36, 48) der Befestigungsvorrichtung (30) und des Sammelbeutels (42) entspricht,
**dadurch gekennzeichnet, dass** die ergänzenden Befestigungsorgane eine Gelenkvorrichtung bilden, die eine distanzierte Schwenkachse (40) der Öffnungen der Befestigungsvorrichtung und des Sammelbeutels eingrenzt, wobei die Gelenkvorrichtung so ausgeführt ist, dass der Beutel, sobald die ergänzenden Befestiqungsorgane zusammenwirken, im Verhältnis zur Befestigungsvorrichtung nur durch Drehen um die besagte Schwenkachse verschoben werden kann, wobei die Schutzfolie so im Verhältnis zur Schwenkachse angeordnet ist, dass sie auch dann noch von der Klebefläche abgezogen werden kann, wenn die ergänzenden Befestigungsorgane bereits zusammenwirken.

2. Anschluss nach Anspruch 1, **dadurch gekennzeichnet, dass** die ergänzenden Befestigungsorgane zumindest zwei Druckknöpfe (38, 52) enthalten, die in einer Reihe angeordnet sind.

3. Anschluss nach Anspruch 1, **dadurch gekennzeichnet, dass** die ergänzenden Befestigungsorgane zwei Elemente (54, 56) enthalten, die jeweils fest an der Befestigungsvorrichtung (30) und am Beutel (42) anhaften, und die eine gegenseitige Befestigung durch magnetische Anziehungskraft bewirken können.

4. Anschluss nach Anspruch 1, **dadurch gekennzeichnet, dass** die ergänzenden Befestigungsorgane eine Lasche (20) enthalten, die fest an einem ersten Anschlussabschnitt anhaftet, sowie einen Bügel (18), der fest am anderen Anschlussabschnitt haftet.

5. Anschluss nach Anspruch 1, **dadurch gekennzeichnet, dass** die ergänzenden Befestigungsorgane geformte Teile (62, 64, 66, 70, 76, 78) aus Stoff mit Ösen und Haken enthalten, die fest an jedem der Anschlussabschnitte (34, 46) haften.

6. Anschluss nach irgendeinem der Ansprüche 1 bis 5, **dadurch gekennzeichnet, dass** die ergänzenden Befestigungsorgane zumindest ein erstes Organ (18) enthalten, das auf einem ersten Anschlussabschnitt angeordnet ist, sowie mehrere zweite Organe (20), die auf dem anderen Anschlussabschnitt angeordnet, und in einem Winkelabstand rund um die Öffnung des entsprechenden Anschlussabschnittes positioniert sind, wobei das erste Organ mit irgendeinem der zweiten Organe zusammenwirken kann.

7. Anschluss nach den Ansprüchen 4 und 6 gemeinsam, **dadurch gekennzeichnet, dass** der Abschnitt, der jenen Teil enthält, der die Lasche (20) trägt, mehrere Laschen aufweist, die in einem Winkelabstand rund um seine Öffnung positioniert sind.

8. Anschluss nach irgendeinem der vorherigen Ansprüche, **dadurch gekennzeichnet, dass** die Schutzfolie (72) der Klebeschicht zumindest zwei Abschnitte enthält, die in Brieftaschenform gefaltet sind, deren Falten so nebeneinander auf der Klebefläche liegen, dass diese Klebefläche vollkommen bedeckt ist, und die Klappe, die nicht auf der Klebefläche haftet eine Lasche (74, 106) enthält, die man ergreifen kann, und die über den Rand der Klebefläche hinausragt.

9. Anschluss nach irgendeinem der vorherigen Ansprüche, **dadurch gekennzeichnet, dass** der Abschnitt mit der Klebefläche dazu bestimmt ist, nach der Verwendung in eine Richtung (T) abgezogen zu werden, und diese Klebefläche so konfiguriert ist, dass die Gesamtlänge der Abziehfläche, die in allgemeiner Richtung senkrecht zur Abziehrichtung liegt, moduliert wird, damit sie auf dem größeren Abschnitt der Länge jenes Teiles etwa konstant ist, auf dem sich die Klebefläche in Abziehrichtung befindet.
